# EUROPEAN PATENT APPLICATION

(11) **EP 4 029 540 A1**
(43) Date of publication of application: **20.07.2022**
(21) Application number: 21202703.1
(22) Date of filing: 14.10.2021
(51) Int. Cl.: A61M 1/06

(54) **A BREAST PUMP**

(30) Priority: 16.12.2020 EP 20214568
(71) Applicant: Koninklijke Philips N.V., 5656 AG Eindhoven (NL)
(72) Inventor: BOURQUIN, Yannyk Parulian Julian, Eindhoven (NL); BROCKHUIS, Lili-Marjan, Eindhoven (NL); WIJNOLTZ, Anna Louise, Eindhoven (NL); LIPSCH, Job, Eindhoven (NL); CLAASSEN, Coen Petrus Martinus, Eindhoven (NL); VAN VELDHUIZEN, Gijsbert Hendrik, Eindhoven (NL)
(74) Representative: Philips Intellectual Property & Standards

(57) **Abstract**

A breast pump has a breast shield for fitting over at least the nipple of a breast thereby to create a cavity over the breast. A stimulation unit is used to apply a physical stimulus to the nipple and/or to a portion of the breast for stimulating the milk ejection reflex. A negative pressure source is used to apply a static negative pressure to the cavity for holding the breast shield on the breast during milk expression, and promoting expression, while milk is collected in a milk collection container. This provides a low cost and low noise breast pump.

## Description

### FIELD OF THE INVENTION

This invention relates to breast pumps.

### BACKGROUND OF THE INVENTION

Breast pumps are used by breast feeding women to extract milk from their breast such that the extracted milk can be fed to their babies at a later time.

It is well known that the best nutrition for babies is breast milk. The world health organization (WHO) recommends to breast feed babies for at least one year, preferably longer. However, mothers often go back to work after only several weeks or months. To provide the best nutrition to their babies, mothers may then express milk using a breast pump. The expressed milk can be stored and given to the baby at a later stage and/or by somebody else.

To use a breast pump, the breast is typically placed into a funnel-shaped cup and a vacuum is applied such that milk is extracted. A motorized breast pump typically has two operating modes, namely a stimulation mode and an extraction mode. The operation of a typical breast pump makes use of a cyclic pressure waveform, typically a negative pressure or vacuum, which is applied to the breast and nipple within a breast shield (or a pair of breast shields) to draw the milk from the nipple into a collection container. The typical pressure profiles oscillate between a maximum negative pressure and then return to atmospheric pressure at the end of each cycle.

During the stimulation mode, the milk ejection reflex is stimulated. This for example makes use of a relatively high frequency cyclic pressure waveform such as 2Hz. Once milk comes out of the breast it is advised to switch to the extraction mode, which has a lower frequency (typically around 1Hz) and higher intensity pumping for more effective milk extraction.

When the breast pump is activated, a pressure source such as a vacuum pump inside the breast pump generates a vacuum and the stimulation mode can be started. The vacuum pump is typically driven by an electric motor. For portable breast pumps, these motors are driven by battery power. There are also breast pumps using manually driven mechanical pumps.

Recently, there is a trend towards wearable breast pumps. These devices are generally in a smaller form factor and can be fully worn on the user's body, allowing the user to move around freely. Some of these are small enough to fit into a user's bra. A motor, battery and milk container are for example integrated to form a single unit.

Additionally, passive milk collectors exist, which can be placed on the breast and often allow the user to apply a static under pressure to hold the device in place and help in extracting milk. These collectors are meant to be used when breastfeeding to collect milk from the non-breastfeeding breast. The stimulation provided by the infant generates a milk ejection reflex on both breasts, and a simple passive vacuum is often enough to extract (some) milk.

In known breast pumps, a particular cyclic pressure waveform is thus used to allow the milk ejection reflex to occur and to extract milk. This requires components such as vacuum pumps, motors, valves, and large batteries. Thus, the device may be expensive, have a very large size, weight, and noise-level.

There is a need for a breast pump with lower price, smaller size and lower noise levels, for example for implementation as a wearable breast pump.

### SUMMARY OF THE INVENTION

The invention is defined by the claims.

According to examples in accordance with an aspect of the invention, there is provided a breast pump, comprising:
a breast shield for fitting over at least the nipple of a breast thereby to create a cavity over at least the nipple;
a stimulation unit for applying a physical stimulus to the nipple and/or to a portion of the breast for stimulating the milk ejection reflex;
a negative pressure source for applying a static negative pressure to the cavity for holding the breast shield on the breast during milk expression for collection in a milk collection container.

It is noted that the static negative pressure may be delivered to the cavity directly or indirectly by means of a membrane. The milk collection container is preferably for receiving milk from the breast shield, but there may instead or additionally be a milk collection chamber at the other breast, in that the milk ejection reflex may trigger milk expression from both breasts.

This breast pump design provides a cost effective, small and quiet way of generating the milk ejection reflex by applying a mechanical oscillation or vibration to the nipple/areola region, in combination with a static negative pressure. The use of a static negative pressure reduces the noise level as well as the power consumption and cost of the components of the system. The static negative pressure may even be mechanically established. A form of stimulation is needed to allow the milk ejection reflex to occur and to extract milk.

The breast pump may fit inside a feeding bra.

The stimulation unit may be a contact stimulation unit.

The contact stimulation unit is for example for applying a physical stimulus to the nipple or areola. Thus, the stimulation provided by the contact stimulation unit is generally in the nipple area. Stimulation may be applied at the tip of the nipple, but this may be too sensitive for some users so that stimulation for a long time may not be desired. The side of the nipple and the areola are less sensitive and can thus be stimulated for a longer period. Stimulation of the nipple top, the nipple side and the areola are all found to be effective in milk supply.

The contact stimulation unit may be for applying a longitudinal or transverse stimulation, relative to the nipple and/or portion of the breast. For example, the stimulation may be longitudinal or transverse relative to a forward axis. This forward axis relates to the direction faced by a user when using the breast pump. It for example corresponds to a length direction of the nipple. The stimulation may thus be in-out with respect to the skin surface or it may be in a direction along the skin surface.

The contact stimulation unit is for example is removably fitted to the breast shield. This enables cleaning of the breast shield with the contact stimulation unit removed.

A stimulation frequency is for example in the range 1 Hz to 1 kHz, such as 2 Hz to 500 Hz, and a stimulation amplitude is for example in the range from 10 µm to 20 mm.

The stimulation settings may be adjustable by a user to obtain the best performance. The adjustment may involve the stimulation pressure and/or the stimulation amplitude and/or the stimulation frequency and/or the stimulation location.

The contact stimulation unit may be adapted to apply the vibrations or oscillations in a pulsed mode or in a continuous mode. A pulsed mode is for example more comfortable particularly for higher frequency vibrations or oscillations.

The contact stimulation unit for example comprises one of:
an electric motor with an eccentric rotating mass or with planetary gears;
a piezoelectric generator;
an electroactive polymer actuator; and
a manual actuator.

These are just some examples of possible way to transmit a force to the breast, in particular the nipple area of the breast.

The contact stimulation unit may comprise a plurality of actuators adapted to be disposed around the nipple (and for example around a portion of the breast).

The stimulation unit may be a non-contact stimulation unit. For instance, the non-contact stimulation unit may be a source of sound waves (for instance a speaker) for generating sound waves for applying a physical stimulus to the nipple or areola. The frequency range of the stimulus may fall in the range of infrasound (<20Hz), which is the inaudible to humans. The waveform of the sound waves can have different shapes or may even be selectable to select the most effective waveform for a user.

In one set of examples, the negative pressure source comprises a manual or electric pump. The noise level will be reduced because a cyclic waveform is not used.

However, in another set of examples, the negative pressure source comprises a compressible chamber for manually creating a reduced pressure in the cavity. The negative pressure source thus delivers a manually-generated reduced pressure, which in particular can function as a baseline pressure for holding the breast pump (i.e. the breast shield) against the breast of the user. This provides a simple solution for generating a holding pressure.

The compressible chamber is preferably elastic and delivers a restoring force to return to a non-compressed state. Thus, when compressed, it is the restoring force of the chamber that creates a pressure reduction in the cavity.

The compressible chamber may comprise the milk collection container. In this case, no additional parts are needed. Instead, the milk collection container has the two functions of generating a baseline under pressure as well as collecting the expressed milk.

The milk collection container for example has a volume greater than 120ml. This is greater than the volume typically needed for milk collection so that an additional volume serves the function of delivering under pressure. Thus, even when all milk has been expressed, the milk collection chamber remains partially compressed and continues to provide a restoring force.

Instead, the breast pump may comprise a separate milk collection container and compressible chamber. In this case, the compressible chamber is used only to create a mechanical under pressure as the static pressure.

The breast pump may further comprise a first, one-way, valve between the breast shield and the milk collection container.

This valve allows milk to flow to the milk collection chamber. The first valve is for example a duckbill valve.

The breast pump preferably further comprises a second, one-way, valve from the compressible chamber to ambient surroundings. When the compressible chamber is compressed air is expelled through the second valve. When the compressible chamber tries to restore its shape, this second valve is closed (because the increase in volume of the compressible chamber creates a pressure drop) and the under pressure in the compressible chamber is thereby retained.

The compressible chamber for example generates a negative pressure between 2kPa and 10kPa (i.e. this amount below atmospheric pressure of around 100kPa). The compressible chamber is for example formed of a rubber material such as silicone.

The breast pump for example comprises a wearable breast pump.

The invention also provides a method of operating a breast pump for the non-therapeutic expression of milk, comprising:
controlling a stimulation unit to apply a physical stimulus to the nipple and/or to a portion of the breast by means of a breast shield, which is fitted over at least the nipple of the breast thereby to create a cavity over at least the nipple, thereby to stimulate the milk ejection reflex; and
controlling a negative pressure source to apply a static negative pressure to the cavity for holding the breast shield on the breast during milk expression.

When the negative pressure source is electrically controlled, rather than manually controlled, the invention also provides a computer program comprising computer program code means which is adapted, when said program is run on processor of the breast pump, to implement the method defined above.

The application of the negative pressure and the stimulation may then be automatically synchronized.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a known breast pump system;
Figure 2 shows a wearable breast pump;
Figure 3 shows one example of a wearable breast pump in accordance with the invention;
Figure 4 shows another example of a wearable breast pump in accordance with the invention;
Figure 5 shows a design of compressible chamber for creating the static pressure;
Figure 6 shows the compressible chamber of the breast pump of Figure 5 being compressed manually; and
Figure 7 shows another example of a wearable breast pump in accordance with the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a breast pump having a breast shield for fitting over at least the nipple of a breast thereby to create a cavity over the breast. A contact stimulation unit is used to apply a physical stimulus to the nipple and/or to a portion of the breast for stimulating the milk ejection reflex. A negative pressure source is used to apply a static negative pressure to the cavity for holding the breast shield on the breast and to assist in milk expression, while milk is collected in a milk collection container. This provides a low cost and low noise breast pump.

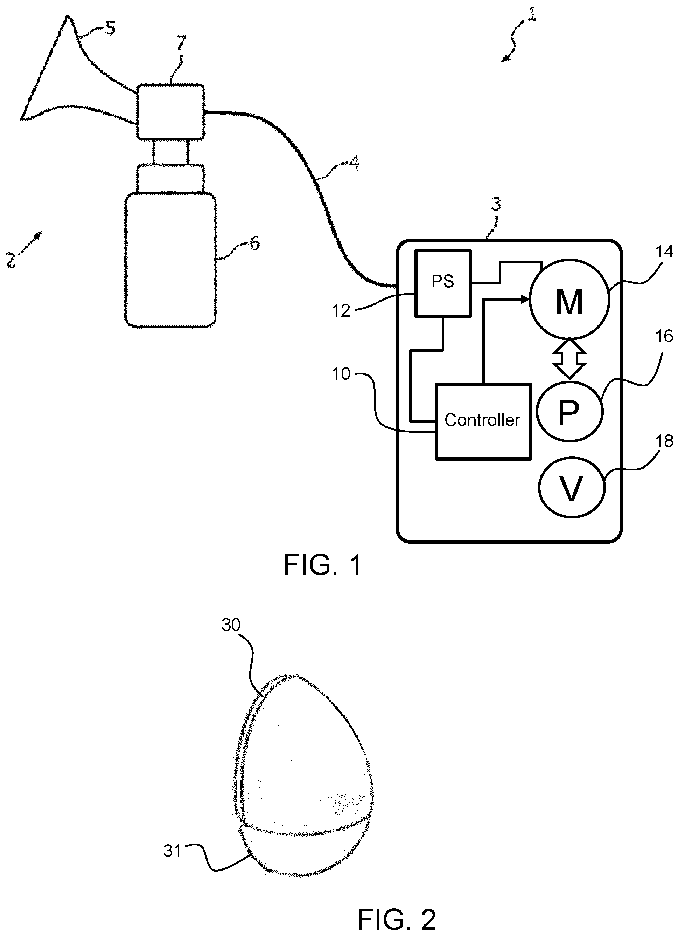

Figure 1 shows a known breast pump system 1, comprising an expression unit 2 and a drive arrangement for controlling the expression function of the breast pump.

In the example shown, the drive arrangement comprises an electric pump arrangement 3 which is connected via a tube 4 to the expression unit 2. The pump arrangement includes various components in addition to a pump impeller and the pump motor, so may be considered to be a general operating unit.

The expression unit 2 is formed with a main body 7, a funnel 5 (known as a breast shield) for receiving a breast of a user and a milk collection container 6 for collecting the expressed milk. The funnel 5 and the container 6 are connected to the main body 7. The main body 7 comprises a vacuum chamber. A flexible membrane known as the diaphragm is located in the vacuum chamber. The diaphragm prevents expressed milk from flowing into the tube 4 leading to the pump arrangement unit 3.

The operating unit, including the pump arrangement 3, may instead be directly mounted and connected to the main body 7. In this case, the membrane prevents expressed milk from flowing directly into the pump arrangement 3.

The pump arrangement 3 comprises a controller 10, a power source 12, a motor 14 and a vacuum pump 16 (i.e. the impeller driven by the motor 14). The controller controls 10 the operation of the power source 12, motor 14 and vacuum pump 16. The pump arrangement 3 further comprises a solenoid valve 18.

In use, the vacuum pump 16 applies a vacuum to the membrane located in the main body 7 so that it deforms. The membrane deforms to create a vacuum in the funnel 5 which in turn applies a vacuum to the breast which enables milk to be expressed.

The vacuum is applied to the breast at intervals. That is, a pressure differential is applied on a cyclic basis. After a vacuum has been established, the pressure from the vacuum is released by the use of the solenoid valve which is temporarily opened. The solenoid valve is an electromechanically operated valve configured to open and close an air passage that connects the vacuum side of the vacuum pump to ambient air such that when the solenoid valve is closed, the vacuum pump generates a vacuum in the expression unit which enables milk to be expressed from the breast of a user. When the solenoid valve is opened, the vacuum generated by the vacuum pump is released as ambient air flows towards the vacuum or negative pressure created by the vacuum pump such that the pressure exerted on the breast of a user is partially or completely reduced.

This is a basic description of the known operation of a standard breast pump system.

There are also wearable breast pumps, which are for example for mounting within a feeding bra. All of the drivetrain components described above are then formed within an outer enclosure which fits over the breast. A top part contains the drivetrain (pump, motor, controller) and a bottom part forms the collection container. This enables breast pumping to be performed more discretely.

There are also other types of wearable breast pump whereby the expression unit is fixed to a breast and the pump unit and/or milk container are situated elsewhere on the body of a user.

Figure 2 shows a wearable breast pump, comprising an expression kit 30 attached to a respective milk collection container 31.

The invention provides a simpler system for stimulating the milk ejection reflex and performing collection of expressed milk. In particular, the invention combines a contact stimulation unit to stimulate the milk ejection reflex with a more simple negative pressure source which applies a static negative pressure.

By static pressure is meant that only a single pressure level is applied and there is no active pressure modulation over time. The pressure may however change over time due to pressure leakage or the filling of the milk container, but there is no active modulation of an applied pressure.

The invention is of particular interest for a wearable breast pump, but it may be applied to other types of breast pump as outlined above to improve ease of use.

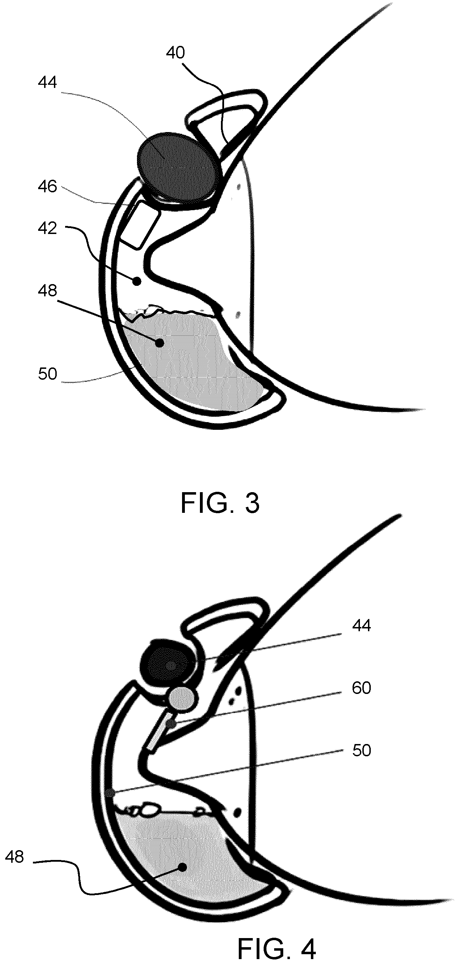

Figure 3 shows a wearable breast pump, comprising a breast shield 40 for fitting over at least the nipple of a breast thereby to create a cavity 42 over the breast. A contact stimulation unit 44 is provided for applying a physical stimulus to the nipple and/or to a portion of the breast for stimulating the milk ejection reflex.

A negative pressure source (shown schematically as 46) is also provided for applying a static negative pressure to the cavity 42 for holding the breast shield on the breast during milk expression and to assist with milk expression. The milk 48 is collected in a milk collection container 50. The container may be rigid or it may be made of a soft material. It for example has a volume between 10 ml to 300 ml, preferably 20 ml to 130 ml. The container and the breast shield unit may be one piece, or the two parts may be attachable and detachable using a click-on method or a screw-on method.

The negative pressure source 46 may comprise a pump (electrical or mechanical) or it may be a compressible chamber for manually creating an under pressure, or it may even be implemented by the milk collection container 50 itself.

The milk ejection reflex is triggered using a mechanical stimulation, such as a vibration or oscillation, on the nipple/areola region to enable milk collection either with a passive or active vacuum.

The contact stimulation unit 44 is for example removably fitted to the breast shield. This for example enables thorough cleaning of the milk contacting parts, for example washing the breast shield in a dishwasher.

There may be a single actuator forming the contact stimulation unit or there may be an array of actuators forming the contact stimulation unit. In an example, the positioning of the actuator or the multiple actuators may be adjusted by the user so that the user can more precisely position the stimulation to be applied to a preferred area. The applied pressure level may also be adjustable, for example by setting a spring bias.

From a study on volunteers, it has been found out that a mechanical oscillation or vibration (i.e. an intermittently applied contact force, or contact force with a cyclic depth of tissue movement and/or applied force) applied to the nipple/areola is sufficient to trigger a milk ejection reflex that is strong enough to extract milk from the breast with a passive vacuum.

The stimulus will simply be referred to as a vibration from this point. Vibration frequencies in the range from 2 to 500 Hz, such as 2 to 250 Hz, and vibration amplitudes in the range from 10 µm to 20 mm have been found to be effective.

The mechanical vibration is applied to the nipple area. From the study with volunteers, the stimulation of the nipple tip has been found to be very effective, but also very sensitive so that stimulation was not comfortable for a long time. The side of the nipple and the areola are less sensitive and could be stimulated for a longer period. Stimulation of the nipple tip, the nipple side and the areola are however all effective in triggering a milk supply.

The system may be tunable to apply different frequencies and amplitudes of vibration, or there may be a cyclic operation including different frequencies and amplitudes of vibration. For example, a cycle may comprise periods with a low frequency and high amplitude and periods with a high frequency and low amplitude. The breast pump may have pre-stored settings which are provided to the user, with different frequencies or amplitudes or combinations of thereof. The vibration can be started and stopped manually with an on/off button.

The vibration may have a vibration axis in a transverse or longitudinal direction (relative to the nipple axis) or a combination of both. The vibration may applied in a continuous mode or in a pulsed mode (wherein each pulse comprises a set of vibrations). A pulsed mode may be perceived as more comfortable when high frequency vibrations are used.

The contact stimulation unit 44 may have any suitable design. It may be electric or mechanical, and may be a linear actuator or a rotary actuator. Some possible examples are:
a motor with an eccentric rotating mass;
a motor actuating planetary gears;
a motor with a cam or similar arrangement for applying a varying contact depth or an intermittent contact and release;
a piezoelectric generator;
an electroactive polymer actuator;
a linear resonant actuator.

The contact stimulation unit could instead use a hydraulic or pneumatic actuator.

The motor in the example above may be replaced with a manually actuated rotating unit. Similarly, instead of an electric motor, a spring powered mechanism may be used.

The negative pressure source 46 is shown only schematically in Figure 3. It is used to create a (partial) vacuum (i.e. a pressure below atmospheric pressure) in the cavity in order to attach the device to the breast and in order to extract milk. It may be a simple pump and impeller, but without needing the hardware and software to generate a cyclic waveform.

A static pressure level (relative to ambient pressure) may be used of -1kPa to -40kPa, for example -2 kPa to -10 kPa.

The negative pressure source may for example comprise a pump as explained above, and optionally also a release valve for adjusting the level of the negative pressure. For example, the pressure may be affected by the filling of the milk collection container with milk, in which case the pump can be used to adjust the pressure. If the negative pressure is felt to be too strong by the user, the valve can be opened by the user (or a command can be provided for the system to open the valve) in order to release the pressure.

Figure 4 shows a modification in which the contact stimulation unit 44 is a motor that is connected to stimulation fingers 60 which better target the nipple and enhance the stimulation. The fingers 60 can apply both low frequency high amplitude motion and high frequency low amplitude vibration. The user can choose to have both types of stimulation at the same time or choose a single type of stimulation.

Figure 4 shows no separate negative pressure source because the milk collection container 50 is used as the negative pressure source.

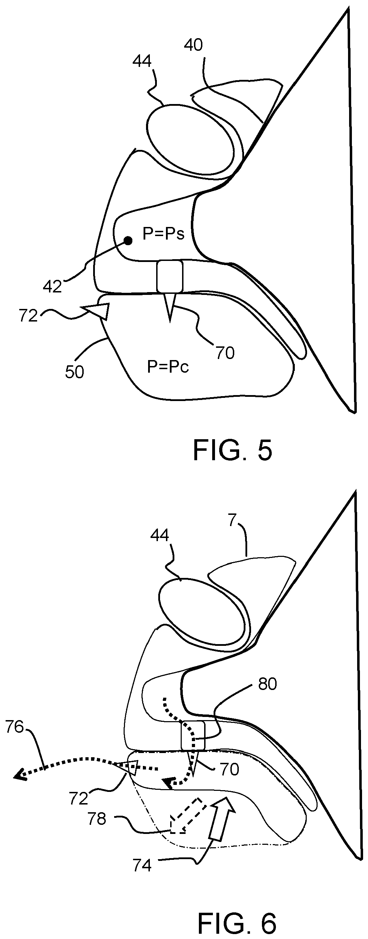

Figure 5 is used to explain this way to use the milk collection container as the negative pressure source in more detail. The milk collection container 50 is in the form of a compressible collapsible chamber for manually creating a reduced pressure in the pressure-controlled cavity 42 of the breast pump.

The compressible chamber 50 is elastic so that it delivers a restoring force to return to a non-compressed state. Thus, when compressed, a restoring force is established, and this creates a pressure reduction in the collapsible chamber 50 and in turn in the cavity 42. The compressible chamber 50 is for example formed of a rubber material such as silicone.

A first, one-way, valve 70 is provided between the breast shield 40 and the milk collection container 50. This first valve 70 allows milk to flow from the cavity 42 to the milk collection container 50. The first valve 70 is for example a duckbill valve. This valve also allows an under pressure to be transferred to the cavity 42 by suction from the compressible chamber 50. Thus, if the pressure in the cavity 42 is higher than in the compressible chamber 50, the valve will open and equalize the pressures.

The breast pump comprises a second, one-way, valve 72 from the compressible chamber 50 to ambient surroundings.

Figure 6 shows the compressible chamber 50 being compressed manually, i.e. by the user performing a squeezing action represented by arrow 74. When the compressible chamber is compressed, air is expelled through the second valve 72 as shown by arrow 76. When the compressible chamber tries to restore its shape by the restoring force, represented by arrow 78, this second valve 72 is closed (because the increase in volume in the compressible chamber 50 creates a pressure drop).

The under pressure in the compressible chamber 50 is thereby retained and it equalizes with the cavity 42 as shown by arrow 80. This manually generated reduced pressure functions as a baseline pressure for holding the breast pump against the breast of the user. This provides a simple solution for generating a holding pressure which also promotes milk expression without using a cyclic pressure waveform.

Before applying the breast pump to the breast, the pressure in the compressible chamber 50 (chamber pressure Pc) is equal to the pressure in the cavity 42 (the breast shield pressure Ps) and equal to atmospheric pressure (Ps=Pc=atmospheric pressure).

When applying the device after squeezing the compressible chamber 50, the chamber pressure Pc and the shield pressure Ps drop such that Pc = Ps < Atm.

The air from the compressible chamber 50 container escapes from the second valve 72.

As the milk container fills, the under pressure may reduce. The user can at any time re-squeeze the compressible chamber 50 to readjust the level of negative pressure. In doing so, the air escapes only from the second valve 72 and does not enter the cavity through the first valve 70.

In this example, the compressible chamber 50 is the milk collection container. Thus, no significant additional parts are needed to create the static under pressure and the milk collection container has the two functions of generating a baseline under pressure as well as collecting the expressed milk.

The milk collection container for example then has a volume greater than 120ml. This is greater than the volume typically needed for milk collection so that an additional volume serves the function of delivering under pressure. Thus, even when all milk has been expressed, the milk collection chamber remains partially compressed and continues to provide a restoring force. If the volume of the compressible chamber container is too low, the pressure Pc may drop too much when the container is filled with milk until a point that the level of under pressure is no longer able to old the device on the breast.

However, even in this case, the user may re-apply the baseline pressure by squeezing the compressible chamber. Thus, depending on the volumes of the parts (and any valve leakage), the breast pump may be designed such that the user is meant to reapply a squeezing pressure at various intervals, or else the initial fitting may be sufficient for the duration of a milk expression session.

In another set of examples, the breast pump further comprises a milk collection container, additional to the compressible chamber. In this case, the compressible chamber is used only to create the static under pressure. The milk collection container may in that case be a rigid container.

In the example above, the valves 70 and 72 are passive valves, so they simply open when the correct pressure differential is present. The valves may instead be controllable, e.g. electrically switchable into an open and/or closed state. In this way, the pressure in the compressible chamber 50 and/or the cavity 42 may be dynamically controllable to maintain a desired static pressure. Pressure sensors may be used to implement a feedback control approach.

The valves may be only actively controlled, or only passively, or they may be passive valves but which can be overridden into a closed and/or open state regardless of the prevailing pressures.

In other examples, the static pressure may be created by an electric or mechanical pump. When a pump is used, it may communicate directly with the cavity 42 or a membrane may separate the cavity 42 from the pump, and the under pressure is generated by a membrane moved by the motor.

There are other ways to create the static under pressure, such as using compressed air or by using an actuator (such as an electroactive polymer) to compress a compressible chamber in the manner explained above instead of having a manually compressible chamber.

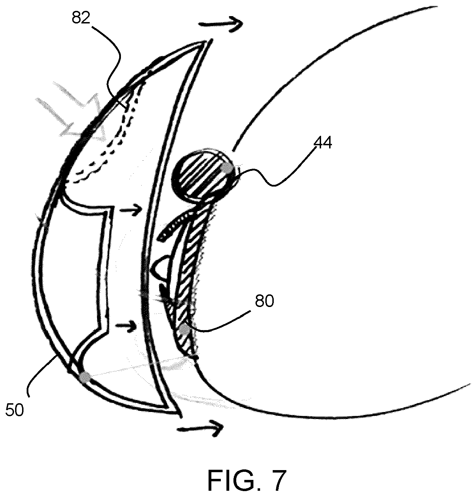

Figure 7 shows an example in which the contact stimulation unit 44 and the milk collection container 50 are separated from each other. They may be used on separate breasts or on the same breast. The contact stimulation unit 44 is held on the breast using a sticky surface. The contact stimulation unit 44 further has a ring 80 that can be placed around the nipple/areola to allow stimulation of a large area around the nipple. Figure 7 shows a compressible portion 82 of the milk collection container 50 to implement the static pressure in the manner explained above.

The contact stimulation unit 44 may then be used alone (with no milk collection container) prior to, or while an infant is breast feeding to help in triggering the milk ejection reflex.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A breast pump, comprising:
a breast shield (40) for fitting over at least the nipple of a breast thereby to create a cavity (42) over at least the nipple;
a stimulation unit (44) for applying a physical stimulus to the nipple and/or to a portion of the breast for stimulating the milk ejection reflex;
a negative pressure source (46) for applying a static negative pressure to the cavity for holding the breast shield on the breast during milk expression for collection in a milk collection chamber (50).

2. The breast pump of claim 1, wherein the stimulation unit is a contact stimulation unit.

3. The breast pump of claim 2, wherein the contact stimulation unit (44) is for applying a physical stimulus to the nipple or areola.

4. The breast pump of claim 2 or 3, wherein the contact stimulation unit (44) is for applying a longitudinal or transverse vibration, relative to said nipple and/or portion of the breast.

5. The breast pump of any one of claims 2 to 4, wherein the contact stimulation unit (44) is removably fitted to the breast shield.

6. The breast pump of any one of claims 2 to 5, wherein a stimulation frequency is in the range 1 Hz to 1 kHz.

7. The breast pump of any one of claims 2 to 6, wherein a stimulation amplitude is in the range from 10 µm to 20 mm.

8. The breast pump of any one of claims 2 to 7, wherein the contact stimulation unit (44) has a user-adjustable:
stimulation pressure; and/or
stimulation amplitude; and/or
stimulation frequency; and/or
stimulation location.

9. The breast pump of any one of claims 2 to 8, wherein the contact stimulation unit (44) is adapted to apply the stimulation in:
a pulsed mode; or
a continuous mode.

10. The breast pump of any one of claims 2 to 9, wherein the contact stimulation unit (44) comprises one of:
an electric motor with an eccentric rotating mass or with planetary gears;
a piezoelectric generator;
an electroactive polymer actuator;
a linear resonant actuator; and
a manual actuator.

11. The breast pump of any one of claims 2 to 10, wherein the contact stimulation unit (44) comprises a plurality of actuators adapted to be disposed around the nipple.

12. The breast pump of any one of claims 2 to 11, wherein the negative pressure source (46) comprises:
a manual or electric pump; or
a compressible chamber (50) for manually creating a reduced pressure in the cavity.

13. The breast pump of any one of claims 1 to 12, comprising a wearable breast pump.

14. A method of operating a breast pump for the non-therapeutic expression of milk, comprising:
controlling a stimulation unit to apply a physical stimulus to the nipple and/or to a portion of the breast by means of a breast shield (40), which is fitted over at least the nipple of the breast thereby to create a cavity (42) over at least the nipple, thereby to stimulate the milk ejection reflex; and
controlling a negative pressure source to apply a static negative pressure to the cavity for holding the breast shield on the breast during milk expression.

15. A computer program comprising computer program code means which is adapted, when said program is run on processor of the breast pump of any one of claims 1 to 10 and in which the negative pressure source comprises a pump, to implement the method of claim 14.
